# EUROPEAN PATENT APPLICATION

(11) **EP 3 431 049 A1**
(43) Date of publication of application: **23.01.2019**
(21) Application number: 16894602.8
(22) Date of filing: 21.12.2016
(51) Int. Cl.: A61F 2/82

(54) **STENT**

(30) Priority: 16.03.2016 JP 2016053080
(71) Applicant: Terumo Kabushiki Kaisha, Tokyo 151-0072 (JP)
(72) Inventor: TANI, Kazuyoshi, Ashigarakami-gun Kanagawa 259-0151 (JP); SAITO, Noboru, Ashigarakami-gun Kanagawa 259-0151 (JP); KOMATSU, Tomoya, Ashigarakami-gun Kanagawa 259-0151 (JP); KUMAZAWA, Takashi, Fujinomiya-shi Shizuoka 418-0015 (JP); UEDA, Ryosuke, Fujinomiya-shi Shizuoka 418-0015 (JP)
(74) Representative: Casalonga
(86) International application number: PCT/JP2016/088237
(87) International publication number: WO 2017/158985

(57) **Abstract**

[Problem] Provided is a stent capable of suppressing formation of a thrombus caused by disturbance in blood flow. [Means for Resolution] There is provided a stent 100 including linear struts 110 that form an outer periphery of a cylindrical shape in which a gap is formed. Each strut has a first convex portion 130 that protrudes inward in a radial direction R in at least a portion in a cross section along an axial direction of the cylindrical shape, and, in a state in which the stent is indwelled in a lumen in a living body, the first convex portion 130 has a vertex 132a that is formed to be positioned on an upstream side of blood flow than a downstream side of blood flow and an upstream curved portion 132 that curves and extends to protrude inward in the radial direction from the vertex toward the upstream side.

## Description

### Technical Field

The present invention relates to a stent.

### Background Art

A stent, for example, is indwelled in an expanded state in a stenosed site or an occlusion site formed in a blood vessel and maintains an open state of the blood vessel. The stent has linear struts, which form an outer periphery of a cylindrical shape in which a gap is formed.

As such a stent, generally, a stent in which a cross section of the struts is formed in a rectangular shape is used. However, in a case where the stent in which a cross section of struts is formed in a rectangular shape is indwelled in the blood vessel, there is a concern that blood flow in a vicinity of the struts is disturbed by a wall on an upstream side of the struts and blood does not flow well.

Regarding this matter, for example, in PTL 1 described below, a stent including a shape (shape of isosceles triangle) that has a slope with respect to a blood flow direction as a cross-sectional shape of the struts is disclosed. According to the stent having such a configuration, since a slope is provided on an upstream side and a downstream side of the blood flow direction, it is possible to improve the blood flow in the vicinity of the struts, and to suppress formation of a thrombus around the struts.

### Citation List

### Patent Literature

PTL 1: JP-A-2015-177956

### Summary of Invention

### Technical Problem

However, in the stent disclosed in PTL 1, because the blood flow is separated from vertexes after the blood flows to the vertexes of the isosceles triangle, there is a possibility that disturbance (vortex) in the blood flow is caused and the thrombus is formed.

From this viewpoint, there is a room for improvement in the stent disclosed in PTL 1 in terms of suppressing formation of the thrombus.

An object of the present invention is to provide a stent capable of suppressing formation of a thrombus caused by disturbance in blood flow. Solution to Problem

According to the present invention for achieving the above-described object, there is provided a stent including linear struts that form an outer periphery of a cylindrical shape in which a gap is formed, in which each strut has a convex portion that protrudes inward in a radial direction in at least a portion in a cross section along an axial direction of the cylindrical shape, and, in a state in which the stent is indwelled in a lumen in a living body, the convex portion has a vertex that is formed to be positioned on an upstream side of blood flow than a downstream side of blood flow and an upstream curved portion that curves and extends to protrude inward in the radial direction from the vertex toward the upstream side. Advantageous Effects of Invention

In a case where the stent that has the above-described configuration is inserted into a blood vessel, the curved shape of the upstream curved portion of the convex portion is disposed along the blood flow direction. For this reason, the blood flows along the upstream curved portion. Here, since the upstream curved portion curves and extends to protrude inward in the radial direction, the blood gradually flows in a direction along the blood flow direction toward the vertex. Therefore, the separation of the blood from the vertex reduces. In addition, since the vertex is provided at the upstream side, it is possible to lengthen the length of the downstream side from the vertex. Therefore, compared with a case where the length of the downstream side is respectively short, since the blood flows more smoothly along the convex portion, it is possible to improve the blood flow in the vicinity of the stent. From the above, it is possible to provide a stent capable of reducing a size of a vortex, and suppressing the formation of a thrombus caused by disturbance in the blood flow in the downstream vicinity of the vertex.

### Brief Description of Drawings

[Fig. 1] Fig. 1 is a perspective view of a stent of an embodiment.
[Fig. 2] Fig. 2 is a developed view in which a part of the outer periphery of the stent of the embodiment is developed by being linearly cut along an axial direction.
[Fig. 3] Fig. 3 is a cross-sectional view taken along line 3-3 of Fig. 2 that is along an axial direction of a bent portion.
[Fig. 4] Fig. 4 is an enlarged view of a portion A of Fig. 2.
[Fig. 5] Fig. 5 is a cross sectional view taken along line 5-5 of Fig. 4 that is along an axial direction of a main strut portion.
[Fig. 6] Fig. 6 is a cross sectional view taken along line 6-6 of Fig. 4 that is along a width direction of the main strut portion.
[Fig. 7] Fig. 7 is a cross sectional view taken along line 7-7 of Fig. 2 that is along an axial direction of a link portion.
[Fig. 8] Fig. 8 is a view for illustrating an action and an effect of the stent of the embodiment. Fig. 8(A) shows blood movement in the vicinity of the stent of the embodiment. Fig. 8(B) shows blood movement in the vicinity of a stent of a comparative example.
[Fig. 9] Fig. 9 is a graph that explains a preferable range of a ratio between an upstream length and a downstream length.
[Fig. 10] Fig. 10 is a cross sectional view corresponding to Fig. 3 of a stent according to Modification Example 1.
[Fig. 11] Fig. 11 is a cross sectional view corresponding to Fig. 3 of a stent according to Modification Example 2.

### Description of Embodiments

Hereinafter, an embodiment of the present invention will be described with reference to the accompanying drawings. Note that, dimensional ratios in the drawings are exaggerated and are different from the actual ratios for the convenience of description.

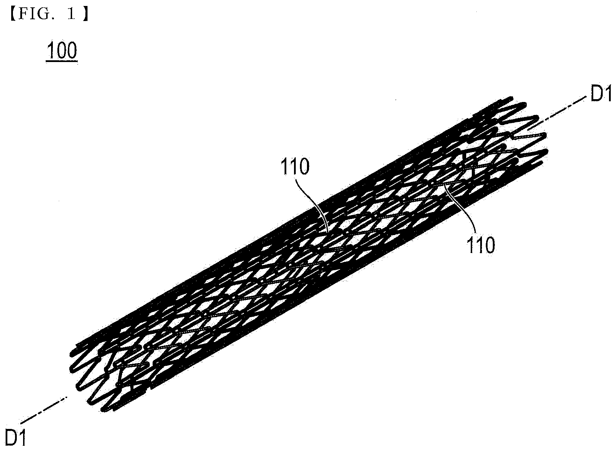

Figs. 1 to 7 are schematic views showing a structure of a stent 100 of the embodiment. Hereinafter, the stent 100 of the embodiment will be described with reference to Figs. 1 to 7.

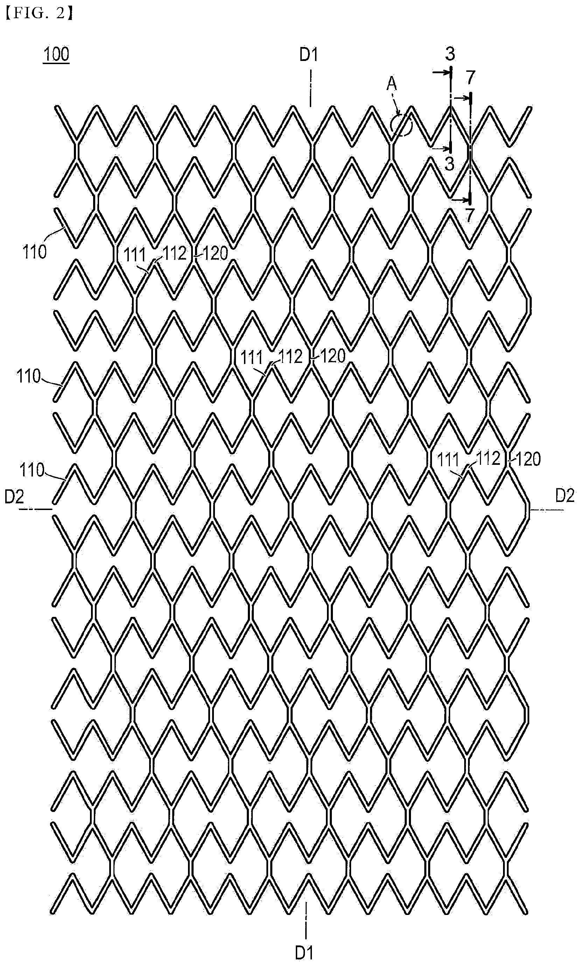

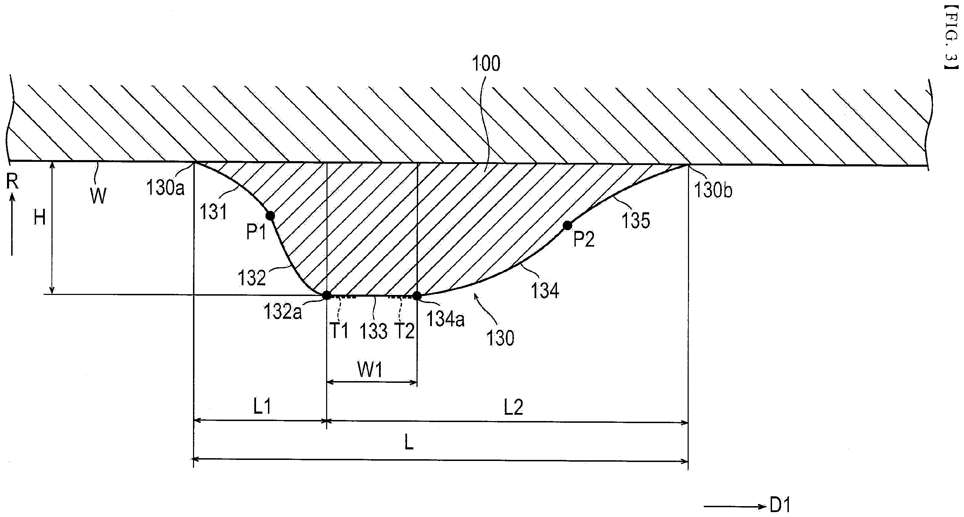

As shown in Figs. 1 and 2, the stent 100 of the embodiment has struts 110, which are linear components, and a plurality of link portions 120 connecting the struts 110 to each other. Note that, in the specification, an axial direction of a cylindrical shape formed using the struts 110 is simply denoted as an "axial direction D1" (refer to Figs. 1 and 2), a circumferential direction of the cylindrical shape is simply denoted as a "circumferential direction D2" (refer to Fig. 2), a radial direction of the cylindrical shape is simply denoted as a "radial direction R" (refer to Figs. 3 and 5 to 7). In addition, a side inserted into the blood vessel is denoted as a "distal side", and a hand operation side that is opposite to the distal side is denoted as a "proximal side". In addition, a direction of the blood flow in a location where the blood flow is not affected by the stent 100 is denoted as a blood flow direction, and in a case where the stent 100 is indwelled in the blood vessel, the blood flow direction coincides with the axial direction D1.

The struts 110 form an outer periphery of the cylindrical shape in which a gap is formed, as shown in Fig. 1. As shown in Fig. 2, the struts 110 have a plurality of main strut portions 111 and a plurality of bent portions 112 interlocking the main strut portions 111 extending in directions different from each other. The struts 110 extend in the circumferential direction D2 while being folded back in a wave shape to form an endless tubular shape.

The plurality of the struts 110 are provided along the axial direction D1 as shown in Fig. 2. The plurality of struts 110 provided along the axial direction D1 are connected to each other through the link portions 120.

The link portions 120 connect the struts 110 to each other (bent portions 112 to each other) in a gap between the struts 110 adjacent along the axial direction D1 as shown in Fig. 2.

The link portions 120 according to the present embodiment are provided along the axial direction D1. Note that, the link portions 120 may be provided along a direction intersecting with the axial direction D1.

The plurality of link portions 120 are disposed at predetermined intervals in the circumferential direction D2. Note that, the location where the link portions 120 are disposed is not limited to the location shown in Fig. 2 and can be appropriately changed as long as the plurality of struts 110 provided along the axial direction D1 are connected to each other.

In the stent 100, the struts 110 and the link portions 120 are integrally formed.

The material forming the stent 100 is, for example, a biodegradable material which is degraded in vivo. Examples of such a material include biodegradable synthetic polymeric materials such as polylactic acid, polyglycolic acid, lactic acid-glycolic acid copolymers, polycaprolactone, lactic acid-caprolactone copolymers, glycolic acid-caprolactone copolymers, and poly-γ-glutamic acid, biodegradable natural polymer materials such as collagen, biodegradable metallic materials such as magnesium and zinc.

The manufacturing method of the stent 100 is not particularly limited, but, examples thereof include a method of cutting out from a tube made of the above-described materials with laser or the like, a method by an injection molding, a method (laminate molding) of laminating using a 3D printer or the like. From a viewpoint of precisely finishing a complicated cross sectional shape, a method by an injection molding and a method of laminating using a 3D printer are preferable. Furthermore, from a viewpoint of manufacturing at a low cost and a viewpoint of finishing as a smooth surface condition, a method by an injection molding is particularly preferable.

In addition, the stent 100 may include a coating body (not shown) containing a medicine on its surface. The coating body is formed on an outer surface side of the stent 100 which is to be come into contact with a blood vessel wall, but the present invention is not limited thereto.

The coating body contains a medicine capable of suppressing proliferation of neointima, and a medicine carrier for carrying the medicine. Note that the coating body may be formed of only the medicine. The medicine contained in the coating body is at least one selected from the group consisting, for example, of sirolimus, everolimus, zotarolimus, and paclitaxel. The constituent material of the medicine carrier is not particularly limited, but a biodegradable material is preferable, and the same material as that of the stent 100 is applicable.

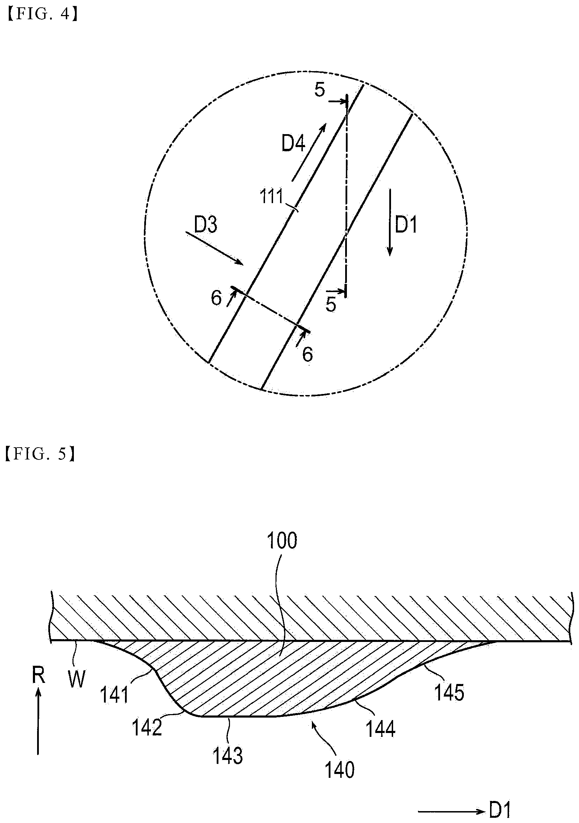

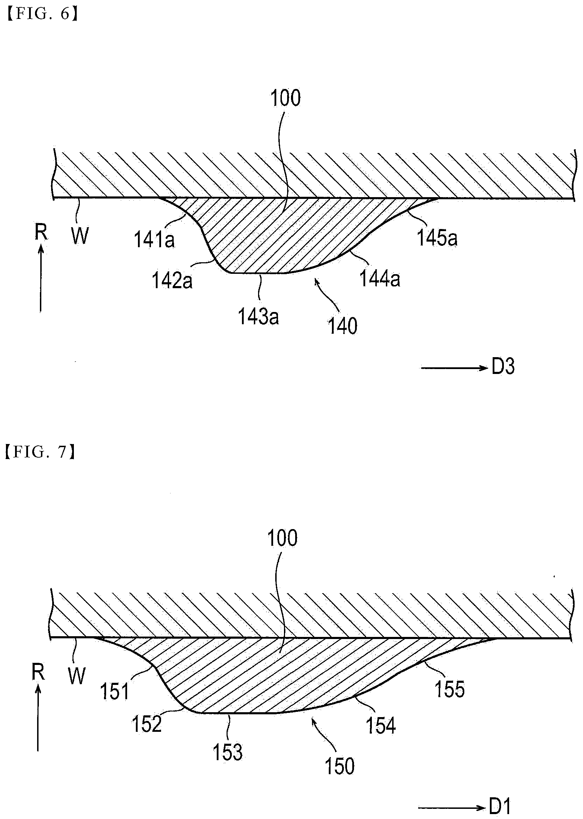

Next, a cross-sectional shape of the stent 100 will be described with reference to Figs. 3 to 7. Note that, in Figs. 3 and 5 to 7, a state where the stent 100 is compressed to a blood vessel wall W is shown. In addition, in each drawing, an upper side of the drawing is the blood vessel wall W side, and a lower side thereof is a blood flow side. Fig. 3 is a cross-sectional view that is taken along line 3-3 of Fig. 2 that is taken along the axial direction D1 of the bent portions 112. Fig. 4 is an enlarged view of a portion A of Fig. 2. Fig. 5 is a cross sectional view taken along line 5-5 of Fig. 4 that is along the axial direction D1 of the main strut portion 111. Fig. 6 is a cross sectional view taken along line 6-6 of Fig. 4 that is along a width direction D3 of the main strut portion 111. Fig. 7 is a cross sectional view taken along line 7-7 of Fig. 2 that is along the axial direction D1 of a link portion 120.

The stent 100 includes a first convex portion 130 (corresponds to convex portion) provided in the bent portions 112 of the struts 110, a second convex portion 140 (corresponds to convex portion) provided in the main strut portions 111 in the struts 110, a third convex portion 150 (corresponds to convex portion) provided in the link portions 120. The first convex portion 130, the second convex portion 140, and the third convex portion 150 protrude inward in a radial direction R, and have a streamline shape so as to improve the blood flow on an upstream side and a downstream side of the blood flow.

Note that, it is preferable that the convex portion is provided in the bent portions 112 and the link portions 120 where the structure is relatively complicated and a vortex is likely to occur. As the convex portion is provided between the bent portions 112 and the link portions 120, it is possible to reduce the vortex size in the bent portions 112 and the link portions 120 and to suppress the formation of a thrombus in the vicinity of the convex portion.

First, a configuration of the first convex portion 130 provided in the bent portions 112 will be described with reference to Fig. 3.

The first convex portion 130 includes an upstream continuous portion 131, an upstream curved portion 132, a linear portion 133, a downstream curved portion 134, and a downstream continuous portion 135 provided in order from the upstream side (left side of Fig. 3) of the blood flow in a cross section along the axial direction D1 as shown in Fig. 3. The upstream continuous portion 131, the upstream curved portion 132, the linear portion 133, the downstream curved portion 134, and the downstream continuous portion 135 are provided continuously. Note that, it is preferable that the above-described each portion is smoothly continuous at an interlocking point of each other from the viewpoint of suppressing the blood separation. In the present specification, "smoothly" means a state without stages and edges at the interlocking point.

The upstream continuous portion 131 curves to be recessed outward (upper side of Fig. 3) in the radial direction R. In addition, when the stent 100 is indwelled in the blood vessel, it is preferable that the upstream continuous portion 131 is smoothly continuous with respect to the blood vessel wall W.

The upstream curved portion 132 curves to protrude inward (lower side of Fig. 3) in the radial direction R. The upstream curved portion 132 and the linear portion 133 are continuous at a vertex 132a.

The vertex 132a is positioned on the upstream side than the downstream side as shown in Fig. 3. That is, when a length from an upstream edge 130a of the first convex portion 130 to the vertex 132a along the axial direction D1 is L1 (hereinafter, referred to as upstream length L1) and a length from a downstream edge 130b of the first convex portion 130 to the vertex 132a along the axial direction D1 is L2 (hereinafter, referred to as downstream length L2), the upstream length L1 is shorter than the downstream length L2.

The linear portion 133 is provided along a tangent line T1 at the vertex 132a of the upstream curved portion 132. The linear portion 133 is provided substantially parallel to the axial direction D1. That is, the linear portion 133 is provided substantially parallel to the blood flow direction.

It is preferable that a length W1 along the axial direction D1 of the linear portion 133 is not more than half of a length L (hereinafter, referred to as total length L) from the upstream edge 130a of the first convex portion 130 to the downstream edge 130b. With such a configuration, it is possible to restrain the length of the first convex portion 130 along the axial direction D1, and form a desired gap between the struts 110.

The downstream curved portion 134 curves to protrude inward in the radial direction R. In addition, the downstream curved portion 134 is configured such that a tangent line T2 in an end portion 134a of the upstream side of the downstream curved portion 134 extends along the linear portion 133.

The downstream continuous portion 135 curves to be recessed outward in the radial direction R. In addition, when the stent 100 is indwelled in the blood vessel, it is preferable that the downstream continuous portion 135 is smoothly continuous with respect to the blood vessel wall W.

It is preferable that the total length L of the first convex portion 130 is longer than a height H along the radial direction R. With such a configuration, it is possible to make an incline in a streamline shape of the first convex portion 130 to be relatively smooth, and the blood flows more smoothly along the first convex portion 130. For this reason, it is possible to improve the blood flow, and to suppress the formation of a thrombus.

Next, a configuration of the second convex portion 140 provided in the main strut portions 111 will be described with reference to Figs. 4 to 6. Note that, since the configuration of the second convex portion 140 is substantially the same as the configuration of the first convex portion 130, a detailed description will be omitted.

The second convex portion 140 includes an upstream continuous portion 141, an upstream curved portion 142, a linear portion 143, a downstream curved portion 144, and a downstream continuous portion 145 provided in order from the upstream side (left side of Fig. 5) of the blood flow in a cross section along the axial direction D1 as shown in Fig. 5. The upstream continuous portion 141, the upstream curved portion 142, the linear portion 143, the downstream curved portion 144, and the downstream continuous portion 145 are provided continuously.

In addition, the second convex portion 140 includes the upstream continuous portion 141a, the upstream curved portion 142a, the linear portion 143a, the downstream curved portion 144a, and the downstream continuous portion 145a provided in order from left side of Fig. 6 in a cross section along a width direction D3 as shown in Fig. 6.

As is known from Figs. 5 and 6, when the cross section along the width direction D3 in the main strut portions 111 of the struts 110 has a uniform streamline shape along an extending direction D4 of the main strut portions 111, a cross section along the axial direction D1 in the main strut portions 111 of the struts 110 also has the same streamline shape.

Next, a configuration of the third convex portion 150 provided in the link portions 120 will be described with reference to Fig. 7. Note that, since the configuration of the third convex portion 150 is substantially the same as the configuration of the first convex portion 130, a detailed description will be omitted.

The third convex portion 150 includes an upstream continuous portion 151, an upstream curved portion 152, a linear portion 153, a downstream curved portion 154, and a downstream continuous portion 155 provided in order from the upstream side (left side of Fig. 7) of the blood flow in a cross section along the axial direction D1 as shown in Fig. 7. The upstream continuous portion 151, the upstream curved portion 152, the linear portion 153, the downstream curved portion 154, and the downstream continuous portion 155 are provided continuously.

Next, the action and the effect of the stent 100 of the present embodiment will be described.

The stent 100 is delivered to a stenosed site or an occlusion site formed in a blood vessel using medical equipment for stent delivery such as a balloon catheter. At this time, such as a coronary stent, in the vicinity of the blood vessel where the stent is indwelled, in a case where the stent is delivered from the upstream side to the downstream side of the blood flow, the downstream side of the stent is disposed on a distal side of the medical equipment for stent delivery and the upstream side of the stent is disposed on a hand operation side.

In this case, a direction of the stent when the stent in the blood vessel is indwelled is positioned such that the upstream side of the stent is positioned on the upstream side and the downstream side of the stent is positioned on the downstream side along the blood flow direction.

Note that, the direction for disposing the stent in the medical equipment for stent delivery is not only set based on the blood flow direction when delivering the stent, but is also preferably set in the consideration of the type of the blood vessel or running of the blood vessel in which the stent is indwelled.

The delivered stent 100 expands in accordance with widening of a balloon in a stenosed site or an occlusion site within a blood vessel. Note that, the stent 100 may be a self-expanding type.

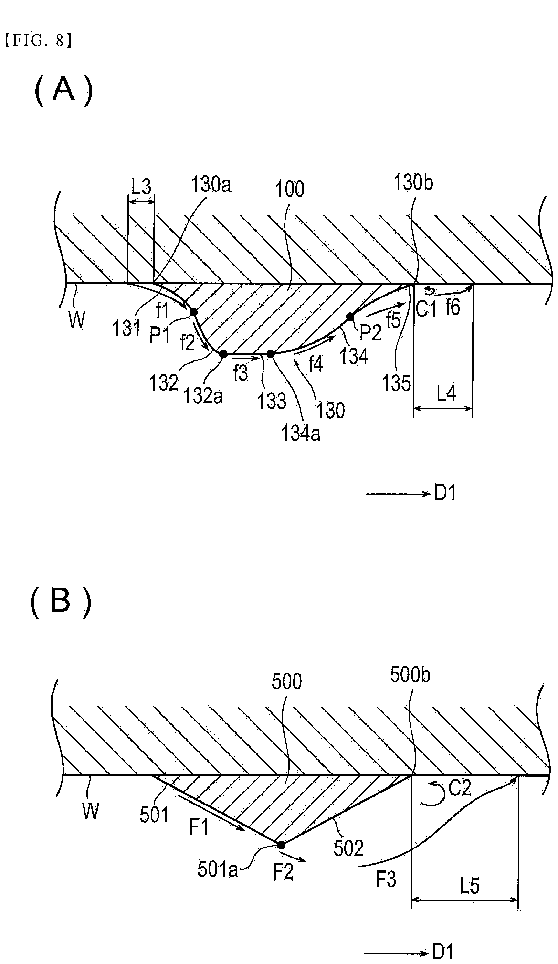

Hereinafter, blood flow of the stent 100 according to the embodiment and a stent 500 according to a comparative example will be compared with reference to Fig. 8 (A) and Fig. 8(B).

First, blood flow in a vicinity of the blood vessel wall W in a case where the stent 500 of the comparative example having a triangular cross section is indwelled in an expanded state in the blood vessel will be described with reference to Fig. 8(B).

In the stent 500 of the comparative example, first, blood flows to a vertex 501a (see symbol F1) along a linear slope 501 of the upstream side of an isosceles triangle, and is separated along an extension line of the linear slope 501 of the upstream side of an isosceles triangle (see symbol F2) . Then, the blood readheres on the blood vessel wall W at a position away from a downstream edge 500b of the isosceles triangle by a length L5 to the downstream side in the axial direction D1. At this time, on the downstream side of the stent 500, a vortex C2 is formed depending on the length L5.

Next, blood flow in a vicinity of the blood vessel wall W in a case where the stent 100 according to the embodiment is indwelled in an expanded state in the blood vessel will be described with reference to Fig. 8(A). Here, blood flow in a vicinity of the first convex portion 130 will be described as an example.

First, the blood flowed to the vicinity of the first convex portion 130 is separated from the blood vessel wall W at a position away from the upstream edge 130a of the first convex portion 130 by a length L3 (hereinafter, referred to as separation length L3) on the upstream side, and flows along the upstream continuous portion 131 (see symbol f1). Here, in order to be smoothly continuous with respect to the blood vessel wall W, it is possible to reduce the size of a vortex of the vicinity of the upstream edge 130a of the first convex portion 130, and to suppress the formation of a thrombus in the upstream continuous portion 131.

Next, blood flows an interlocking point P1 that interlocks the upstream continuous portion 131 and the upstream curved portion 132. Here, since the upstream continuous portion 131 and the upstream curved portion 132 are smoothly continuous at the interlocking point P1, it is possible to suppress the blood separation at the interlocking point P1. Accordingly, it is possible to suppress the formation of a thrombus at the interlocking point P1.

Then, the blood flows along the upstream curved portion 132 and flows to the vertex 132a (see symbol f2). At this time, the blood is slightly separated from the first convex portion 130 in the vicinity of the most distant location from a straight line that links the interlocking point P1 and the vertex 132a in the upstream curved portion 132. Here, since the upstream curved portion 132 curves and extends to protrude inward in the radial direction R, the direction of blood flow in the vicinity of the first convex portion 130 gradually comes along with the axial direction D1 (blood flow direction) toward the vertex 132a. Therefore, the separation of blood from the vertex 132a reduces compared to the stent 500 (see Fig. 8 (B)) including the linear slope 501 on the upstream side. For this reason, it is possible to reduce the size of the vortex on the downstream side of the vertex 132a, and to suppress the formation of a thrombus.

Next, the blood flows along the linear portion 133 (see symbol f3). Here, since the linear portion 133 is provided parallel to the axial direction D1 (blood flow direction), even the blood slightly separated in the vicinity of the most distant location from a straight line that links the interlocking point P1 and the vertex 132a in the upstream curved portion 132 gradually flows along the axial direction D1 (blood flow direction) in the linear portion 133. Therefore, it is possible to reduce the size of a vortex at the vicinity of the linear portion 133, and to suppress the formation of a thrombus.

Next, the blood flows the end portion 134a of the upstream side of the downstream curved portion 134. Here, since the linear portion 133 and the downstream curved portion 134 are smoothly continuous in the end portion 134a of the downstream curved portion 134, it is possible to suppress the separation of blood from the first convex portion 130 in the end portion 134a. Therefore, it is possible to suppress the formation of a thrombus in the vicinity of the end portion 134a.

Next, the blood flows along the downstream curved portion 134 (see symbol f4). Here, since the downstream curved portion 134 extends to protrude inward in the radial direction R, the blood flows along the downstream curved portion 134 compared to the stent 500 (see Fig. 8(B)) including a linear slope 502 on the downstream side (compare symbol f4 in Fig. 8(A) symbol F3 in Fig. 8(B)). Therefore, it is possible to suppress the separation of blood from the downstream curved portion 134, and to suppress formation of a thrombus in the vicinity of the downstream curved portion 134.

Next, the blood is gradually separated from the first convex portion 130 starting from the vicinity of the most distant location from a straight line that links the end portion 134a and the interlocking point P2 of the downstream curved portion 134 and the downstream continuous portion 135 in the downstream curved portion 134 (see symbol f5).

Next, the blood readheres on the blood vessel wall W at a position away from a downstream edge 130b of the first convex portion 130 by a length L4 (hereinafter, referred to as readhesion length L4) to the downstream side in the axial direction D1. At this time, on the downstream side of the first convex portion 130, a vortex C1 is formed depending on the length L4.

Since the stent 100 according to the present embodiment includes the first convex portion 130, the readhesion length is short compared to the stent according to the comparative example with reference to Fig. 8(A) and Fig. 8(B). Then, due to short readhesion length, stagnation on the downstream side of the stent 100 is suppressed, and the size of the vortex C1 is reduced. Therefore, according to the stent 100 according to the embodiment, compared with the stent 500 according to the comparative example, it is possible to suppress the formation of a thrombus on the downstream side of the stent 100.

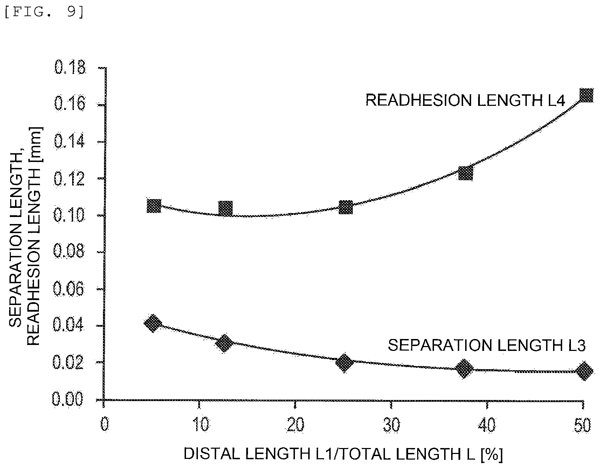

Next, a preferred range of a ratio between the upstream length L1 and the downstream length L2 will be described with reference to Fig. 9. Fig. 9 shows simulation results of a fluid analysis.

In Fig. 9, a horizontal axis shows a ratio of the upstream length L1 with respect to the total length L. In addition, between vertical axes, diamond-shaped plots indicate separation length L3, and rectangular plots indicate readhesion length L4, respectively.

It is preferable that the separation length L3 is 0.03 mm or less from a viewpoint of suppressing the formation of a thrombus in the vicinity of the upstream edge 130a. Therefore, it is preferable that the ratio of the upstream length L1 with respect to the total length L is 12.5% (1/8) or more based on the graph in Fig. 9.

On the other hand, it is preferable that the readhesion length L4 is 0.125 mm or less from a viewpoint of suppressing the formation of a thrombus in the vicinity of the downstream edge 130b. Therefore, it is preferable that the ratio of the upstream length L1 with respect to the total length L is 37.5% (3/8) or less based on the graph in Fig. 9.

Therefore, it is preferable that the ratio of lengths between the upstream length L1 and the downstream length L2 is within a range of 1:7 to 3:5.

As described above, the stent 100 of the present embodiment is the stent 100 including the linear struts 110 that form an outer periphery of a cylindrical shape in which a gap is formed. Each strut 110 includes the first convex portion 130 that protrudes inward in the radial direction R in at least a portion in a cross section along the axial direction D1 of the cylindrical shape. In a state where the stent 100 is indwelled in a lumen in a living body, the first convex portion 130 includes the vertex 132a that is formed to be positioned on the upstream side of blood flow than the downstream side of blood flow and the upstream curved portion 132 that curves and extends to protrude inward in the radial direction R from the vertex 132a toward the upstream in a cross section along the axial direction D1 of the cylindrical shape.

In a case where the stent 100 having the above-described configuration is inserted into a blood vessel, a curved shape of the upstream curved portion 132 of the first convex portion 130 is disposed along the blood flow direction. For this reason, the blood flows along the upstream curved portion 132. Here, since the upstream curved portion 132 curves and extends to protrude inward in the radial direction R, the direction of blood flow in the vicinity of the upstream curved portion 132 gradually comes along with the blood flow direction toward the vertex 132a. Therefore, the separation of the blood from the vertex 132a reduces. In addition, since the vertex 132a is provided on the upstream side of the first convex portion 130, it is possible to lengthen the length of the downstream side from the vertex 132a. Accordingly, compared with a case where the length to the downstream side is respectively short, since the blood flows more smoothly along the first convex portion 130, it is possible to improve the blood flow in the vicinity of the stent 100. From the above, in the vicinity of the downstream side of the vertex 132a, it is possible to reduce the size of a vortex and to suppress the formation of a thrombus caused by disturbance in the blood flow.

In addition, the first convex portion 130 is provided on the downstream side from the vertex 132a in a cross section along the axial direction D1, and further includes the downstream curved portion 134 that curves and extends to protrude inward in the radial direction. For this reason, the blood flows along the downstream curved portion 134 more compared to the stent 500 including the slope 502 on the downstream side. Therefore, it is possible to improve the blood flow, and to suppress the formation of a thrombus in the vicinity of the downstream curved portion 134.

In addition, the first convex portion 130 is configured so that the ratio of the upstream length L1 and the downstream length L2 is within a range of 1:7 to 3:5 in a cross section along the axial direction D1. According to the configuration, as described above, it is possible to set the separation length L3 and the readhesion length L4 to be a desired length, and to suitably suppress the formation of a thrombus.

In addition, the first convex portion 130 further includes the linear portion 133 that interlocks the upstream curved portion 132 and the downstream curved portion 134, and is provided along a tangent line in the vertex 132a of the upstream curved portion 132, in a cross section along the axial direction D1. For this reason, the blood slightly separated from the vertex 132a gradually flows along the axial direction D1 (blood flow direction) in the linear portion 133. Accordingly, it is possible to reduce the size of a vortex in the vicinity of the linear portion 133.

In addition, the first convex portion 130, in a cross section along the axial direction D1, further includes the upstream continuous portion 131 that is continuous to the upstream edge 130a of the first convex portion 130 from the end portion (interlocking point P1) of the upstream side in the upstream curved portion 132 and curves to be recessed outward in the radial direction R, and the downstream continuous portion 135 that is continuous to the downstream edge 130b of the first convex portion 130 from the end portion (interlocking point P2) of the downstream side in the downstream curved portion 134 and curves to be recessed outward in the radial direction R. Therefore, when the stent 100 is indwelled in the blood vessel, the upstream continuous portion 131 and the downstream continuous portion 135 are smoothly continuous with respect to the blood vessel wall W. Therefore, the blood flow in the upstream edge 130a and the downstream edge 130b of the first convex portion 130 improves, and it is possible to reduce the size of a vortex in the upstream edge 130a and the downstream edge 130b of the first convex portion 130, and to suppress the formation of a thrombus.

In addition, the stent 100 further includes the plurality of link portions 120 that connects the struts 110 with each other in a gap. The struts 110 include the plurality of main strut portions 111 and the plurality of bent portions 112 that include the main strut portions 111 extending in directions different from each other. Each bent portion 112 includes the first convex portion 130, and each link portion 120 includes the third convex portion 150. Accordingly, since the first convex portion 130 and the third convex portion 150 are provided respectively in the bent portions 112 and the link portions 120 where the structure is comparatively complicated and a vortex is likely to occur, it is possible to reduce the size of vortex in the bent portions 112 and the link portions 120, and to suppress the formation of a thrombus.

### <Modification Example 1 of Convex Portion>

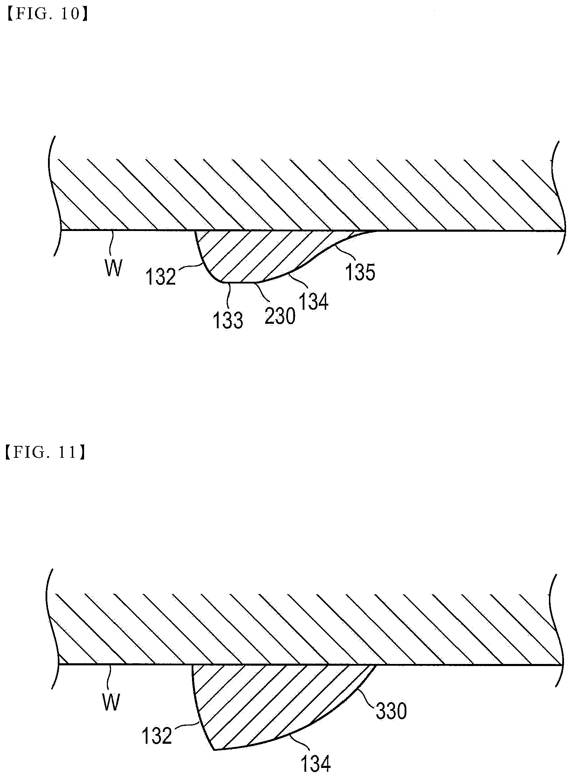

The first convex portion 130 according to the above-described embodiment had the upstream continuous portion 131, the upstream curved portion 132, the linear portion 133, the downstream curved portion 134, and the downstream continuous portion 135. However, a shape of a first convex portion is not particularly limited as long as the upstream curved portion 132 is provided, and the vertex 132a on the downstream side of the upstream curved portion 132 is positioned on the downstream side than the upstream side. For example, a configuration in which the upstream continuous portion 131 is not provided and the upstream curved portion 132, the linear portion 133, the downstream curved portion 134, and the downstream continuous portion 135 are provided may be adopted as the first convex portion 230 in Fig. 10.

### <Modification Example 2 of Convex Portion>

In the above-described embodiment, the first convex portion 130 had the upstream continuous portion 131, the upstream curved portion 132, the linear portion 133, the downstream curved portion 134, and the downstream continuous portion 135. However, a configuration that includes only the upstream curved portion 132 and the downstream curved portion 134 without the upstream continuous portion 131, the linear portion 133, and the downstream continuous portion 135 may be adopted as shown in the first convex portion 330 in Fig. 11.

### <Other Modification Examples>

In the above-described embodiment, the second convex portion 140 is provided in the main strut portions 111. However, the main strut portions may have a rectangular cross section. In the case of a stent with such a configuration, since the cross sections at locations other than the bent portions 112 and the link portions 120 become a rectangular shape, and the area in a cross sectional view increases compared to a streamline shape, when the stent expands, it is possible to impart a desired expansion force with respect to the blood vessel wall W, and to suitably maintain the open state of the blood vessel. In addition, since the first convex portion 130 and the third convex portion 150 are provided respectively in the bent portions 112 and the link portions 120 where a vortex easily occurs, it is possible to suppress formation of a thrombus.

The present invention is not limited to the embodiment and the modification examples described above, and can be variously modified within the claims.

For example, in the above-described embodiment, the link portions 120 are integrally configured with the struts 110. However, the link portions 120 may be configured separately from the struts 110. At this time, the link portions 120 are configured with, for example, a biodegradable material. On the other hand, the struts 110, for example, may be configured with a non-biodegradable material. Examples of such a material include stainless steel, a cobalt-based alloy such as a cobalt-chromium alloy (for example, a CoCrWNi alloy), elastic metal such as a platinum-chromium alloy (for example, a PtFeCrNi alloy), and a super-elastic alloy such as a nickel-titanium alloy.

In addition, in the above-described embodiment, the stent 100 is configured such that the plurality of struts 110 are continuous in the axial direction D1. However, the strut of the stent may be configured in a spiral shape.

In addition, in the above-described embodiment, the first convex portion 130, the second convex portion 140, and the third convex portion 150 have a substantially same shape. However, the first convex portion 130, the second convex portion 140, and the third convex portion 150 may have different shapes as long as the upstream curved portions 132, 142, and 152 are provided.

In addition, even when the above-described convex portion is formed along the axial direction D1 before expansion deformation of the stent 100, as long as the above-described convex portion is formed along the axial direction D1 after the expanded deformation of the stent 100, it is included in the present invention.

This application is based on Japanese Patent Application No. 2016-053080 filed on March 16, 2016, the disclosure of which is incorporated by reference in its entirety.

### Reference Signs List

100 stent
110 strut
111 main strut portion
112 bent portion
120 link portion
130, 230, 330 first convex portion (convex portion)
140 second convex portion (convex portion)
150 third convex portion (convex portion)
130a upstream edge
130b downstream edge
131, 141, 151 upstream continuous portion
132, 142, 152 upstream curved portion
133, 143, 153 linear portion
134, 144, 154 downstream curved portion
135, 145, 155 downstream continuous portion
D1 axial direction
L1 upstream length (length along axial direction from upstream edge of convex portion to vertex)
L2 downstream length (length along axial direction from downstream edge of convex portion to vertex)
P1 interlocking point (end portion on upstream side in upstream curved portion)
P2 interlocking point (end portion on downstream side in downstream curved portion)
R radial direction

## Claims

1. A stent comprising:
linear struts that form an outer periphery of a cylindrical shape in which a gap is formed,
wherein each strut has a convex portion that protrudes inward in a radial direction in at least a portion in a cross section along an axial direction of the cylindrical shape, and
wherein, in a state in which the stent is indwelled in a lumen in a living body,
the convex portion has,
a vertex that is formed to be positioned on an upstream side of blood flow than a downstream side of blood flow, and
an upstream curved portion that curves and extends to protrude inward in the radial direction from the vertex toward the upstream side.

2. The stent according to Claim 1,
wherein, in the cross section along the axial direction,
the convex portion further has,
a downstream curved portion that is provided on the downstream side from the vertex and that curves and extends to protrude inward in the radial direction.

3. The stent according to Claim 2,
wherein, in the cross section along the axial direction, the convex portion is configured so that a ratio of a length along the axial direction from an upstream edge of the convex portion to the vertex and a length along the axial direction from a downstream edge of the convex portion to the vertex is within a range of 1:7 to 3:5.

4. The stent according to Claim 2 or 3,
wherein, in the cross section along the axial direction,
the convex portion further includes,
a linear portion that interlocks the upstream curved portion and the downstream curved portion and is provided along a tangent line at the vertex of the upstream curved portion.

5. The stent according to any one of Claims 2 to 4,
wherein, in the cross section along the axial direction,
the convex portion further includes,
an upstream continuous portion that is continuous from an end portion on the upstream side in the upstream curved portion to the upstream edge of the convex portion and curves to be recessed outward in the radial direction, and
a downstream continuous portion that is continuous from an end portion on the downstream side in the downstream curved portion to the downstream edge of the convex portion and curves to be recessed outward in the radial direction.

6. The stent according to any one of Claims 1 to 5, further comprising:
a plurality of link portions that connects the struts to each other in the gap,
wherein each strut has a plurality of main strut portions and a plurality of bent portions that interlocks the main strut portions that extend in different directions, and
wherein at least one of the plurality of link portions and/or at least one of the plurality of bent portions has the convex portion.

7. The stent according to any one of Claims 1 to 6, which is manufactured by injection molding or laminate molding.
